# FASCICULE DE BREVET EUROPEEN

(11) **EP 1 655 004 B1**
(45) Date de publication et mention de la délivrance du brevet: **10.12.2008**
(21) Numéro de dépôt: 05356195.7
(22) Date de dépôt: 04.11.2005
(51) Int. Cl.: A61F 5/02

(54) **Ceinture lombaire**
Rückengurt
Lumbar support belt

(30) Priorité: 05.11.2004 FR 0411824
(43) Date de publication de la demande: 10.05.2006
(73) Titulaire: Gibaud, 42000 Saint Etienne (FR)
(72) Inventeur: Anquetil, Lionel, 03110 Serbannes (FR)
(74) Mandataire: Delorme, Nicolas

(56) Documents cités:
- US-A- 4 175 553
- US-A- 4 796 315
- US-A- 5 690 609
- US-B1- 6 319 217

## Description

La présente invention concerne une ceinture lombaire.

Une ceinture lombaire est une ceinture médicale réalisée dans une matière textile élastique, qui a pour fonction d'assurer chez le sujet qui la porte un maintien de la région lombaire ainsi que de la région abdominale. Le port d'une ceinture lombaire a pour but de réaliser un rappel de posture pour soulager et rééduque un sujet souffrant de lombalgie aiguë ou chronique.

Le rappel de posture est obtenu par la nervosité du textile ainsi que par des baleines. Ces baleines, qui sont notamment en acier ressort, sont cambrées pour s'adapter à la forme de la partie du corps humain, en l'occurrence la partie inférieure du tronc.

Les ceintures lombaires actuelles donnent globalement satisfaction, cependant sur au moins un point, elles ne le sont pas totalement. En effet, les ceintures actuelles doivent être obligatoirement mises en place selon la même orientation par rapport au patient, la légère concavité de la baleine devant venir suivre la concavité de la région lombaire.

Il est connu par le document US 6 319 217 une ceinture ayant un coussinet conformable qui est inséré dans une poche ménagée dans la ceinture.

On connaît par le document US 5 690 609 une ceinture comprenant des panneaux reliés à des sangles.

Le document US 4 796 315 divulgue un coussin lombaire en mousse maintenu par une sangle pour un port en position assise ou couchée.

Le document US 4 175 553 décrit une ceinture dont l'une des faces reçoit un appareillage orthopédique.

L'invention a pour but de remédier à cet inconvénient et a notamment pour but de proposer une ceinture lombaire qui soit réversible.

L'invention concerne une ceinture lombaire pouvant être mise en place sur un patient comme définie dans la revendication 1.

Pour assurer un effort de rappel élastique le dossard est constitué d'un panneau de textile armé d'au moins une baleine.

De façon préfère, le dossard comprend quatre baleines métalliques ce qui permet d'assurer un excellent rappel de posture.

Dans une mise en oeuvre de la ceinture selon l'invention, la partie antérieure est amovible de la partie postérieure et peut être fixée indifféremment à l'une des extrémités de la partie postérieure.

Pour sa bonne compréhension, l'invention est décrite en référence au dessin ci annexé représentant, à titre d'exemple non limitatif, plusieurs formes de réalisation de ceinture lombaire selon celle-ci.

Figures 1 et 2 représentent une première forme de réalisation d'une ceinture selon l'invention dans deux configurations,

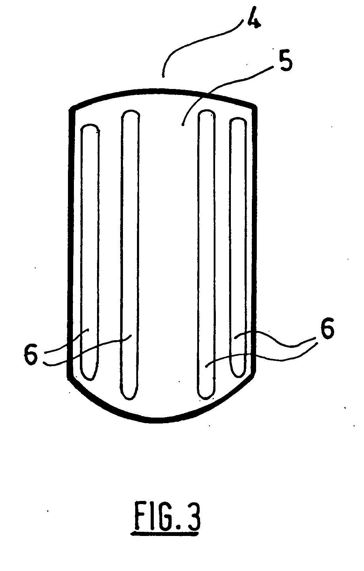

Figure 3 montre une forme d'exécution des moyens de rappel de posture,

Figures 4 et 5 montrent une deuxième forme de réalisation d'une ceinture dans deux configurations,

Figures 6 et 7 montrent une troisième forme de réalisation d'une ceinture selon l'invention dans deux configurations.

En se reportant au dessin, on peut constater que la ceinture 1 est constituée d'une partie postérieure 2 qui peut venir recouvrir la région lombaire d'un patient et une partie antérieure 3 qui peut venir recouvrir la région abdominale du patient.

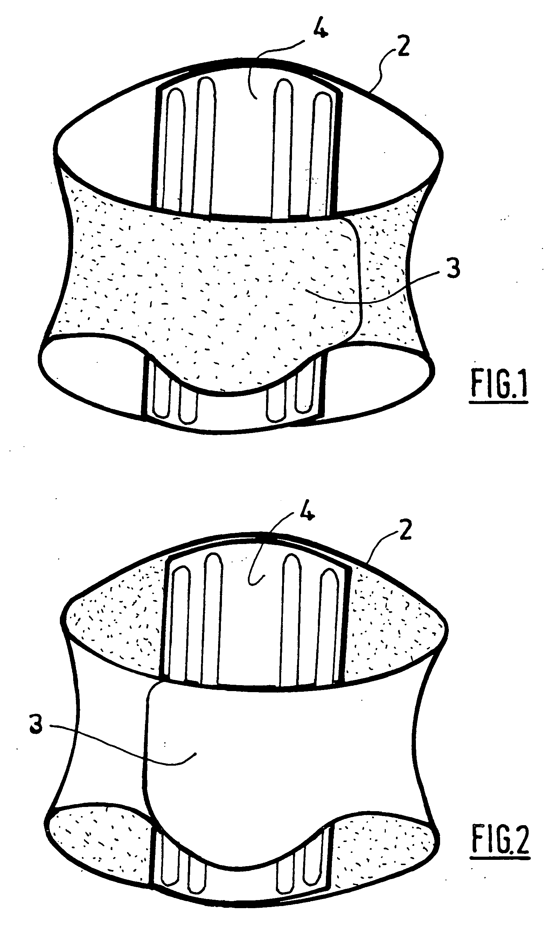

Dans l'exemple représenté aux figures 1 et 2, la ceinture 1 peut utiliser comme moyens de fermeture des rubans de textile à boucles et crochets qui sont, respectivement, cousus sur l'extrémité de la partie postérieure et sur l'extrémité de la partie antérieure.

La particularité de cette ceinture est que ses moyens de rappel de posture sont constitués par un dossard 4 amovible qui peut être, indifféremment, mis en place sur l'une des faces de la partie postérieure 2 de la ceinture de la ceinture. Ainsi, la ceinture est véritablement réversible, puisqu'il suffit d'ôter le dossard 4 amovible de l'une des faces de la partie postérieure 2 pour le mettre en position sur la face opposée.

Le dossard 4 peut est constitué d'un panneau de textile 5 armé de quatre baleines 6 métalliques, chacune des baleines 6 présentant un concavité adaptée aux courbes de la région lombaire.

Grâce à cette disposition, on peut prévoir pour chacune des faces de la ceinture des textiles de nature différente et présentant donc des propriétés de confort ou d'aspect spécifiques.

La fixation du dossard 4 peut être réalisée par un textile agrippant du type boucles et crochets qui est rapporté sur le dossier et sur chacune des faces de la partie postérieure de la ceinture.

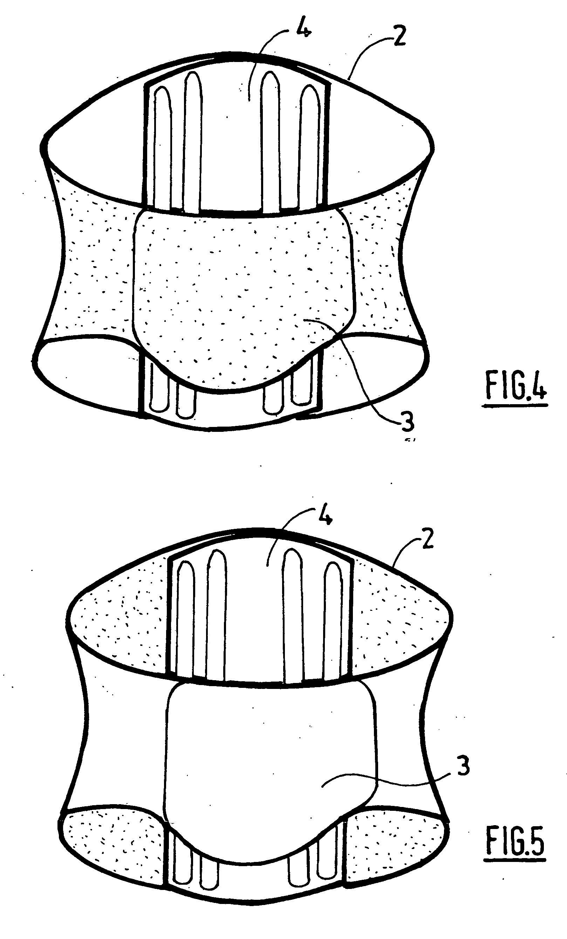

La seconde forme de réalisation de l'invention représentée aux figures 4 et 5 permet de corriger un éventuel défaut de la ceinture réversible selon la première forme de réalisation décrite précédemment. En effet, lorsque la ceinture lombaire change de configuration, en d'autres termes lorsque le dossard est positionné depuis une face à l'autre face de la ceinture, la fermeture de la partie antérieure change de côté pour le patient. Or, pour un patient souffrant de lombalgie, le fait de ne pas utiliser sa main préférentielle, généralement celle de droite, peut aggraver ses douleurs. C'est pour cela que, dans une seconde forme de réalisation de l'invention, il est prévu que la partie antérieure de la ceinture soit elle-même amovible pour que, selon la face qui soit amenée à être en contact du patient, celui-ci puisse toujours choisir une configuration de la ceinture qui lui permette d'effectuer un serrage avec sa main préférentielle.

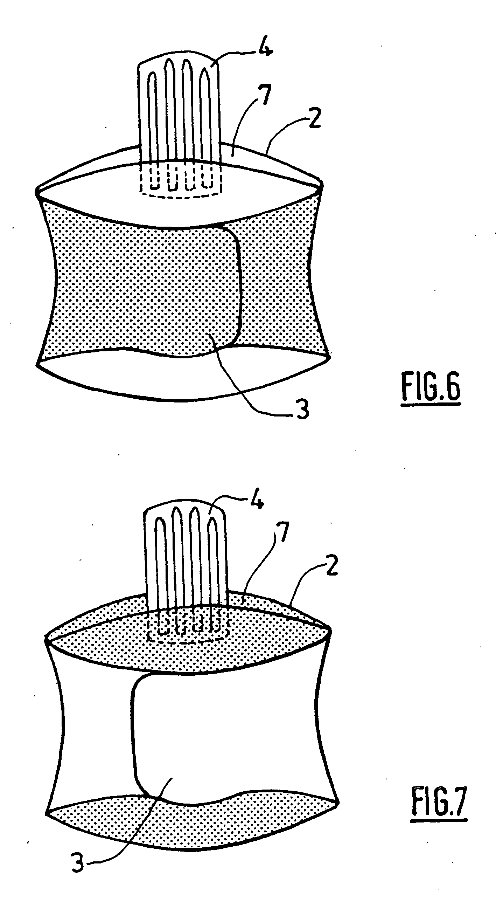

Comme cela apparaît sur les figures 6 et 7, il est également envisagé que la partie postérieure 2 présente une poche centrale 7 dans laquelle le dossard 4 peut être glissé avec sa concavité orientée indifféremment vers l'une des faces de la partie postérieure 2.

Bien entendu, l'invention n'est pas limitée à la forme de réalisation décrite ci-dessus, mais elle est définie par les revendications.

## Revendications

1. Ceinture lombaire pouvant être mise en place sur un patient comportant
- une partie postérieure (2), pouvant couvrir la région lombaire d'un patient, équipée de moyens élastiques de rappel de posture présentant une concavité, et
- une partie antérieure (3) pouvant couvrir la région abdominale du patient, la partie postérieure et la partie antérieure présentant chacune à leur extrémité des moyens de fermeture complémentaires, **caractérisée en ce que** les moyens de rappel de posture sont constitués par un dossard (4) concave amovible pouvant être fixé indifféremment sur l'une des faces de la ceinture, chacune des faces de la partie postérieure (2) est équipée de bandes de textile agrippant et le dossard (4) est équipé de bandes de textile agrippant pouvant venir se fixer indifféremment sur l'une des faces de la partie postérieure (2).

2. Ceinture lombaire selon la revendication 1, **caractérisée en ce que** le dossard (4) est constitué d'un panneau de textile (5) armé d'au moins une baleine (6).

3. Ceinture lombaire selon la revendication 2, **caractérisée en ce que** le dossard comprend quatre baleines (6).

4. Ceinture lombaire selon l'une des revendications 1 à 3, **caractérisée en ce que** la partie antérieure (3) est amovible de la partie postérieure (2) et peut être fixée indifféremment à l'une des extrémités de la partie postérieure.

## Claims

1. Lumbar support belt that can be fitted to a patient, comprising
- a posterior part (2) able to cover the lumbar region of a patient, provided with elastic posture correcting means having a concavity, and
- an anterior part (3) able to cover the abdominal region of the patient, the posterior part and the anterior part each having complementary closure means at their ends, **characterized in that** the posture correcting means consist of a removable concave back piece (4) that can be fixed to either surface of the belt, each surface of the posterior part (2) is provided with gripping textile strips and the back piece (4) is provided with gripping textile strips that can be fixed on either surface of the posterior part (2).

2. Lumbar support belt according to Claim 1, **characterized in that** the back piece (4) consists of a textile panel (5) reinforced with at least one stiffener (6).

3. Lumbar support belt according to Claim 2, **characterized in that** the back piece comprises four stiffeners (6).

4. Lumbar support belt according to one of Claims 1 to 3, **characterized in that** the anterior part (3) can be removed from the posterior part (2) and can be fixed to either end of the posterior part.

## Patentansprüche

1. Lumbalgürtel, der an einem Patienten platziert werden kann, mit
einem hinteren Teil (2), mit dem der Lumbalbereich eines Patienten abgedeckt werden kann und der mit elastischen Haltungskorrekturmitteln, die eine Konkavität aufweisen, ausgestattet ist, und
einem vorderen Teil (3), der den Abdominalbereich des Patienten abdecken kann, wobei der hintere Teil und der vordere Teil jeweils an ihrem Ende komplementäre Schließmittel aufweisen, **dadurch gekennzeichnet, dass** die Haltungskorrekturmittel aus einem entfernbaren konkaven Rückenteil (4) bestehen, das unterschiedslos an einer der Flächen des Gürtels befestigt werden kann, jede der Flächen des hinteren Teils (2) mit Festhaltetextilstreifen ausgestattet ist und das Rückenteil (4) mit Festhaltetextilstreifen ausgestattet ist, die unterschiedslos an einer der Flächen des hinteren Teils (2) befestigt werden können.

2. Lumbalgürtel nach Anspruch 1, **dadurch gekennzeichnet, dass** das Rückenteil (4) aus einem Textilfeld (5) besteht, das mit mindestens einer Versteifung (6) verstärkt ist.

3. Lumbalgürtel nach Anspruch 2, **dadurch gekennzeichnet, dass** das Rückenteil vier Versteifungen (6) umfasst.

4. Lumbalgürtel nach einem der Ansprüche 1 bis 3, **dadurch gekennzeichnet, dass** der vordere Teil (3) vom hinteren Teil (2) entfernbar ist und unterschiedslos an einem der Enden des hinteren Teils befestigt werden kann.
